(19)

Europäisches
Patentamt

European
Patent Office

Office européen
des brevets

(11) **EP 4 105 195 A1**

(12) **EUROPEAN PATENT APPLICATION**
published in accordance with Art. 153(4) EPC

(43) Date of publication:
**21.12.2022 Bulletin 2022/51**

(21) Application number: **21754252.1**

(22) Date of filing: **26.01.2021**

(51) International Patent Classification (IPC):
**C07C 67/48** *(2006.01)*      **C07C 69/612** *(2006.01)*
**B01J 31/02** *(2006.01)*

(52) Cooperative Patent Classification (CPC):
**B01J 31/02; C07C 67/48; C07C 69/612**

(86) International application number:
**PCT/KR2021/001028**

(87) International publication number:
**WO 2021/162284 (19.08.2021 Gazette 2021/33)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **14.02.2020  KR 20200018495**

(71) Applicant: **Hanwha Solutions Corporation
Jung-gu
Seoul 04541 (KR)**

(72) Inventors:
• **PARK, Seongmin**
  **Daejeon 34128 (KR)**
• **KIM, Hyo Suk**
  **Daejeon 34128 (KR)**
• **JUNG, Kitaeg**
  **Daejeon 34128 (KR)**

(74) Representative: **Berggren Oy**
**P.O. Box 16
Eteläinen Rautatiekatu 10A
00101 Helsinki (FI)**

(54) **ESTER-BASED COMPOUND PRODUCTION METHOD**

(57)     This invention relates to a preparation method of an ester compound that can reduce energy consumption due to convenient post-treatment process, and simultaneously, can produce products of high purities.

EP 4 105 195 A1

**Description**

[Technical Field]

Cross-reference to Related Application

This application claims the benefit of Korean Patent Application No. 10-2020-0018495 filed on February 14, 2020 with the Korean Intellectual Property Office, the disclosures of which are herein incorporated by reference in their entirety.

This invention relates to a method for preparing an ester compound that can be used as a plasticizer.

[Background Art]

Ester compounds prepared by the reaction of polycarboxylic acid and alcohol are being widely used as plasticizers.

The esterification reaction process of carobxylic acid and alcohol for obtaining ester compounds is progressed at high temperature in the presence of a titanate catalyst, and after the reaction is completed, in the reaction mixture, a catalyst, catalyst decomposition products, unreacted alcohol and carboxylic acid, monoester, reaction impurities, and the like are included. Thus, in order to obtain pure ester compounds, a post-treatment process is required, and in such a post-treatment process, the steps of neutralization, washing, dealcoholization, and/or filtration, and the like may be included.

In the post-treatment process, the neutralization step neutralizes acidic materials such as residual carboxylic acid and monoester, and the like, and enables hydrolysis of catalyst, and it conducted by introducing base and water in the reaction mixture and reacting them.

However, the temperature of the reaction mixture, immediately after the esterification reaction is finished, is about 220 to 240 °C, and in case a base aqueous solution is immediately introduced in this state, water is evaporated, and thus, hydrolysis of catalyst may not smoothly occur. And, as such, if hydrolysis of catalyst is not smoothly progressed, catalyst decomposition products may be aggregated or gelled, and thus, may be difficult to remove by filtration later.

Thus, in order to solve the problems, a method of cooling the reaction mixture immediately after the reaction to about 100 to 120 °C, and then, conducting a neutralization reaction is being used. However, in this case, in order to remove residual alcohol and water after the neutralization step, the temperature should be increased again to about 150 °C, and thus, both cooling and temperature-raising processes are required in the post-treatment after the reaction, thus rendering the process complicated and increasing energy consumption.

[Detailed Description of the Invention]

[Technical Problem]

In order to solve the problem, it is an object of the invention to provide a method for preparing an ester compound that omits cooling and temperature-raising processes of the post-treatment process during the production process of an ester compound, and thus, can increase energy efficiency, and simultaneously, can obtain high purity products.

[Technical Solution]

According to one embodiment of the invention, there is provided a method for preparing an ester compound comprising steps of:

reacting polycarboxylic acid and alcohol in the presence of a titanium-based catalyst to obtain a reaction mixture comprising an ester compound (step 1);
removing unreacted alcohol from the reaction mixture to 5 wt% or less (step 2);
adding base and water to the reaction mixture from which unreacted alcohol has been removed, and conducting a neutralization reaction and hydrolysis reaction of the titanium-based catalyst at a pressure of 3 bar or more and a temperature of 150 °C or more (step 3);
removing water from the reaction mixture in which the neutralization reaction and hydrolysis reaction have been completed (step 4); and
filtering (step 5).

According to one embodiment, the temperature of the step 2 may be 150 °C to 250 °C.

According to one embodiment, the temperature of the step 3 may be 150 °C to 190 °C.

According to one embodiment, the pressure of the step 3 may be 3 bar to 10 bar.

According to one embodiment, the step 3 may be conducted for 10 minutes or less.

According to one embodiment, the base of the step 3 may be one or more selected from the group consisting of caustic soda(NaOH), caustic potash(KOH), sodium carbonate($Na_2CO_3$), and sodium bicarbonate.

According to one embodiment, the step 4 may be conducted by a decompression stripping process.

According to one embodiment, the polycarboxylic acid may be phthalic acid, isophthalic acid, or terephthalic acid.

According to one embodiment, the alcohol may be C5-20 aliphatic alcohol.

According to one embodiment, the titanium-based catalyst may be one or more selected from the group consisting of tetra isopropyl titanate, tetra n-butyl titanate, tetra octyl titanate and butyl tin maleate.

According to one embodiment, a separate cooling process may not be included between the step 1 to step 3.

According to another embodiment of the invention, there is provided an ester compound prepare by the above preparation method.

The ester compound may have impurity content less than 1.0 wt%.

The ester compound may have an acid value of 0.1 KOHmg/g or less.

According to the invention, there is also provided a plasticizer composition comprising the ester compound.

[Effect of the Invention]

According to the preparation method of an ester compound of the invention, cooling and temperature-raising processes are not necessary in the post-treatment process after esterification, thus remarkably reducing energy consumption of the preparation process, shortening process time, and preparing a high-quality ester compound having high purity. Thus, according to the invention, the production cost of an ester compound used as a plasticizer may be reduced, and production efficiency may be improved.

[Detailed Description of the Embodiments]

The terms used herein are only to explain specific embodiments, and are not intended to limit the invention. A singular expression includes a plural expression thereof, unless it is expressly stated or obvious from the context that such is not intended. As used herein, the terms "comprise", "equipped" or "have", etc. are intended to designate the existence of practiced characteristic, number, step, constructional element or combinations thereof, and they are not intended to preclude the possibility of existence or addition of one or more other characteristics, numbers, steps, constructional elements or combinations thereof.

Although various modifications can be made to the invention and the invention may have various forms, specific examples will be illustrated and explained in detail below. However, it should be understood that these are not intended to limit the invention to specific disclosure, and that the invention includes all the modifications, equivalents or replacements thereof without departing from the spirit and technical scope of the invention.

According to one embodiment of the invention, there is provided a method for preparing an ester compound comprising steps of:

> reacting polycarboxylic acid and alcohol in the presence of a titanium-based catalyst to obtain a reaction mixture comprising an ester compound (step 1);
> removing unreacted alcohol from the reaction mixture to 5 wt% or less (step 2);
> adding base and water to the reaction mixture from which unreacted alcohol has been removed, and conducting a neutralization reaction and hydrolysis reaction of the titanium-based catalyst at a pressure of 3 bar or more and a temperature of 150 °C or more (step 3);
> removing water from the reaction mixture in which the neutralization reaction and hydrolysis reaction have been completed (step 4); and
> filtering (step 5).

In general, ester compounds are produced by reacting carboxylic acid and alcohol, followed by purification through post-treatment processes of neutralization, washing, dealcoholization and filtration of the reaction mixture. However, in this case, a process of cooling the high temperature reaction mixture is required so that water introduced in the neutralization step may not be evaporated, and during the subsequent dealcoholization, the temperature should be increased again to remove alcohol, and thus, energy consumption of the entire process is large and the process is inefficient.

Thus, according to the invention, after esterification, unreacted alcohol is first removed from the reaction mixture, and then, hydrolysis of a titanium-based catalyst and neutralization of acid components are conducted, and the hydrolysis and neutralization reactions are progressed under high temperature high pressure conditions. Thus, a cooling process of the reaction mixture for preventing the evaporation of water in the step of hydrolysis of catalyst and neutralization is not separately required, and hydrolysis of catalyst may be progressed very quickly under high temperature high pressure conditions.

And, since solid hydrolysis products of catalyst and neutralized salts produced in the catalyst hydrolysis and neutralization reactions are easily removed through a filtering process, according to the invention, energy consumption of the preparation process of an ester compound may be reduced and production efficiency may be significantly improved.

Hereinafter, the invention will be explained in detail according to steps.

First, an esterification reaction wherein polycarboxylic acid and alcohol are reacted in the presence of a titanium-based catalyst is conducted (step 1).

The polycarboxylic acid may be aliphatic or aromatic carboxylic acid having 2 or more, specifically 2 to 4 carboxyl groups(-COOH) in the molecule. Or, anhydrides of the polycarboxylic acids may be used.

Specifically, the polycarboxylic acid may be C2 to C20 aliphatic or C6 to C20 aromatic carboxylic acid. For example, the polycarboxylic acid may be one or more selected from the group consisting of adipic acid, azelaic acid, phthalic acid, isophthalic acid, terephthalic acid, citric acid, trimellitic acid, and anhydrides thereof, but is not limited thereto. Preferably, the polycarboxylic acid may be one or more selected from the group consisting of phthalic acid, isophthalic acid, terephthalic acid and anhydrides thereof, and more preferably, terephthalic acid or an anhydride thereof.

The alcohol may be C1 to C20, preferably C5 to C15 linear or branched aliphatic alcohol, and specifically, one or more selected from the group consisting of butanol, hexnaol, 2-ethylhexnaol, isononyl alcohol, isodecyl alcohol, and propyl heptanol.

The preparation method of an ester compound according to one embodiment of the invention may be a method of preparing dioctyl terephthalate (di 2-ethylhexyl terephthalte, DOTP) using terephthalic acid as the polycarboxylic acid and 2-ethyl hexanol as the alcohol.

The titanium-based catalyst used for the esterification reaction is a titanium alkoxide-based catalyst, and tetra isopropyl titanate, tetra n-butyl titanate, tetra octyl titanate, butyl tin maleate, and the like may be used, and they may be used alone or combinations.

The amounts of the polycarboxylic acid, alcohol, and titanium-based catalyst used are not specifically limited herein, and may be appropriately controlled according to the knowledge in the art. And, the reaction conditions of the esterification of polycarboxylic acid and alcohol are not specifically limited, either, but for example, it may be conducted at a temperature of 180 °C to 260 °C and a pressure greater than 1.0 $kg/cm^2$ to 4.0 $kg/cm^2$.

By the above esterification reaction, a reaction mixture of high temperature comprising an ester compound is obtained. In the reaction mixture, besides an ester compound aimed, unreacted polycarboxylic acid, alcohol, acidic impurities such as monoester, catalyst residue, and the like remain, and thus, a post-treatment process for purification is required.

According to one embodiment of the invention, as the first step of such a post-treatment process, a step of removing unreacted alcohol from the reaction mixture is conducted (step 2). Wherein, the step 2 is conducted until the content of unreacted alcohol in 100 wt% of the reaction mixture becomes 5 wt% or less, 1 wt% or less, 0.5 wt% or less, preferably 0.02 wt% or less.

Since unreacted alcohol included in an ester compound causes bad smell, it should be necessarily removed to secure product quality. And, if the content of unreacted alcohol in the reaction mixture is 5 wt% or more, the hydrolysis of catalyst in step 3 may not smoothly occur, and the reaction time may be lengthened, and thus, process efficiency may be lowered.

The step of removing unreacted alcohol may be conducted through a stripping process.

The stripping process may be conduced in a reactor itself, or may be conducted using a distillation column. Wherein, as the distillation column, a packing column, a tray column, and the like may be illustrated, but is not limited thereto, and commonly used distillation columns may be used without limitations.

As the stripping medium, inert gas may be used, and preferably nitrogen may be used. The flow rate of the nitrogen gas may be appropriately controlled according to temperature and pressure conditions during stripping and production scale.

Meanwhile, before the stripping, a process of firstly removing unreacted alcohol through flashing in the front stage of the column may be optionally conducted. The flashing may be conducted at a temperature of 190 to 200 °C and a pressure of 0.5 $kg/cm^2$ or less, or 0.1 $kg/cm^2$ or less, preferably 0.07 $kg/cm^2$ or less.

The step of removing unreacted alcohol is conducted under reduced pressure and high temperature conditions. Specifically, a pressure in the step of removing unreacted alcohol may be 0.5 $kg/cm^2$ or less, or 0.1 $kg/cm^2$ or less, preferably 0.07 $kg/cm^2$ or less. And, the temperature may be in the range of 150 °C to 250 °C, preferably 170 °C to 200 °C. If the temperature of the unreacted alcohol removing step is less than 150 °C, even if a vacuum degree of the column and the flow rate of the stripping medium are controlled, unreacted alcohol may not be sufficiently removed, and thus, alcohol content of the final product may not meet product standard (less than 200 ppm).

As explained above, since the step of removing unreacted alcohol is conducted at a temperature of 150 °C to 250 °C, a separate cooling process of the reaction mixture is not required between the step 1 and step 2. Although the temperature of the reaction mixture, immediately after the esterification reaction of step 1, is about 220 to 240 °C, the temperature may be decreased by about 20 to 30 °C in the process of transferring it to equipment for conducting step 2, thereby meeting the optimum temperature required for the unreacted alcohol removing step.

Meanwhile, in case the preparation method of the invention is continuously conducted, the alcohol component recovered in the step of removing unreacted alcohol may be introduced in the esterification reaction again and reused.

Next, base and water are added to the reaction mixture from which unreacted alcohol has been removed, to neutralize compounds having acid groups such as unreacted polycarboxylic acid and monoester, and the like, and conduct hydrolysis reaction of the titanium-based catalyst (step 3).

Particularly, according to the invention, the neutralization reaction and hydrolysis reaction of catalyst are conducted under high temperature(150 °C or more) and high pressure(3 bar or more) conditions, to remarkably shorten reaction time, thereby increasing efficiency and economic feasibility of the preparation process.

As the base, base effective for neutralization, commonly used in the art, may be used, and specifically, aqueous solutions of caustic soda(NaOH), caustic potash(KOH), sodium carbonate($Na_2CO_3$), and sodium bicarbonate($NaHCO_3$), and the like may be used.

The amount of the neutralization agent used may be appropriately controlled according to the acid value of the reaction solution, and for example, it may be used at the number of moles of 1 to 5 times of the number of moles of unreacted polycarboxylic acid and monoester, on the basis of monovalent base.

Wherein, the number of moles of unreacted polycaboxylic acid and monoester may be calculated through measurement of acid value(KOHmg/g). The acid value is mg of KOH required to neutralize acid contained in 1g of a sample, and is calculated according to the following Equation.

**[Equation 1]**

$$\text{Acid value} = (0.5611 \times a \times f) / S$$

In the Equation,

S is the amount of a sample collected(g),
a is the amount of 0.01N KOH solution consumed(mℓ), and
f is titer of 0.01N KOH solution.

It is preferable that water is introduced at the number of moles of 300 to 800 times, more preferably 300 to 600 times of the number of moles of the titanium-based catalyst used in step 1. If the amount of water introduced is too small, hydrolysis reaction of the catalyst may not sufficiently occur, and if the amount of water introduced is too large, energy consumption for removal of water from the reaction mixture may increase, and waste water generation may increase.

According to the invention, in order to increase the speed of neutralization reaction and hydrolysis of catalyst, the step 3 is conducted under high temperature condition of 150 °C or more. However, under high temperature condition, water required for hydrolysis reaction is evaporated, and thus, in order to prevent this, a reaction pressure is controlled to 3 bar or more. As such, in case neutralization and hydrolysis of catalyst are conducted under high temperature and high pressure conditions, a reaction speed may be remarkably improved, thus increasing process efficiency.

Specifically, the temperature of the step 3 may be 150 °C to 190 °C, or 150 °C to 170 °C, and the pressure may be 3 bar to 10 bar, or 3 bar to 5 bar. Thus, a separate cooling process is unnecessary between the step 2 and step 3.

Under the above temperature and pressure conditions, the neutralization reaction and hydrolysis of catalyst may be completed within 10 minutes, preferably within 5 minutes.

After the step 3, the titanium-based catalyst is converted into solid titanium dioxide($TiO_2$), and acid components are also converted into solid salts, and thus, may be easily removed in the filtering step later.

Next, water is removed from the reaction mixture after completing the step 3 (step 4).

The step 4 may be conducted through a decompression stripping process using a distillation column. Wherein, the distillation column and stripping medium that can be used are as explained in the step 2.

In the step 4, stripping may be conducted under pressure condition of 0.5 kg/cm$^2$ or less, 0.3 kg/cm$^2$ or less, preferably 0.15 kg/cm$^2$ or less, and the feed rate of inert gas may be appropriately controlled according to temperature and pressure conditions during stripping and production scale.

The temperature of the step 4 may be preferably 140 °C to 180 °C, more preferably 150 °C to 170 °C. Within the above temperature range, water in the reaction mixture may be effectively removed.

Next, the reaction mixture, after completing the step 4, filtered to obtain a purified ester compound(step 5). By such a filtering step, impurities such as excessively used neutralization agent in step 3, titanium dioxide, salts, and the like may be removed from the reaction mixture.

Although the filtering method is not specifically limited, it may be conducted by a method of adding filter aid such as diatomaceous earth, activated carbon, silica gel, and the like to the reaction mixture and mixing them, and then, introducing the mixture in a candle filter type solid liquid separation filter(FUNDABAC®) and filtering at 100 to 120 °C for 3 to 4 hours. For a small scaled process, a filter paper may be used.

According to the preparation method of the invention, without needing to cool the high temperature reaction mixture after the esterification reaction, dealcoholization and neutralization, hydrolysis of catalyst may be immediately conducted continuously, and thus, processability may be significantly improved, and energy may be reduced, thereby increasing productivity. The preparation method may be conducted batchwise, semicontinuously or continuously, and applied for the preparation of dibutyl phthalate(DBP), dioctyl terephthalate(DOTP), diisononyl phthalate(DINP), diisodecyl phthalate(DIDP), and the like.

And, the ester compound prepared by the above explained preparation method has low acid value and exhibits high purity, and thus, can be suitably used as the raw material of a plasticizer. Specifically, the ester compound prepared according to the invention may have impurity content less than 1.0 wt% (namely, purity of 99.0 wt% or more), less than 0.5 wt%, or less than 0.2 wt%. The smaller impurity content is preferable, and thus, the lower limit is not limited, but for example, it may be 0.01 wt% or more.

And, the acid value of the ester compound prepared according to the invention may be 0.1 KOHmg/g or less, 0.05 KOHmg/g or less, or 0.01 KOHmg/g or less. The acid value is the weight(mg) of potassium hydroxide(KOH) required to neutralize acid contained in 1g of a sample, and it may be calculated by titration of a sample solution with 0.01N alcoholic KOH solution. The lower the acid value, more excellent the product quality, and thus, the lower limit is not limited, but for example, it may be 0.001 KOHmg/g or more.

The ester compound prepared according to the invention may be suitably used as a plasticizer of resin selected from the group consisting of ethylene vinyl acetate, polyethylene, polypropylene, polyvinyl chloride, polystyrene, polyurethane, polybutadiene, silicone, thermoplastic elastomer or copolymers thereof.

Hereinafter, the invention will be explained in more detail through examples. However, these examples are presented only as the illustrations of the invention, and the detailed description of the invention is not limited thereto. And, in the following Examples and Comparative Examples, "%" and "parts" indicating content are on the basis of weight, unless otherwise described.

[Example]

Example 1

(Step 1) In a reactor, 400g of terephthalic acid(TPA) 1000g of 2-ethylhexanol(2-EH), and 0.8g of tetra n-butyl titante(TBT) were introduced and reacted to prepare dioctyl terephthalate(DOTP). Wherein, the esterification reaction temperature was 180 to 230 °C, and the pressure was 1 to 1.5 kg/cm$^2$.

After the esterification reaction was completed, the reaction mixture discharged from the reactor comprised about 75 wt% of DOTP, about 25 wt% of 2-EH, small amounts of the catalyst and unreacted TPA, monoester, and the like.

(Step 2) Using a batch reactor, while flowing nitrogen at 180 °C, 0.05 kg/cm$^2$ at the rate of 500 cc/min, stripping was progressed for 1 hour to obtain a reaction mixture in which 2-EH was completely removed.

(Step 3) To the reaction mixture, distilled water(20 g) was introduced at the number of moles of 500 times of the number of moles of the catalyst used for the esterification reaction, and NaOH was introduced as a neutralization agent at the number of moles of 4 times of the number of moles of unreacted TPA and mono-ester, and the mixture was stirred at a temperature of 170 °C and a pressure of 5 bar for 5 minutes to progress hydrolysis of the catalyst and neutralization of acid components.

(Step 4) And then, using a batch reactor, while flowing nitrogen at 180 °C, 0.05 kg/cm$^2$ at a rate of 500 cc/min, stripping was progressed for 10 minutes to remove water.

(Step 5) The reaction mixture, after stripping had been completed, was cooled to 120 °C, and diatomaceous earth was introduced, and then, a filtering process was conducted using a filter paper to prepare dioctyl terephthalate.

**Example 2**

Dioctyl terephthalate was prepared by the same method as Example 1, except that the temperature of the neutralization and catalyst hydrolysis step(step 3) was 150 °C, and the pressure was 3 bar.

6

### Example 3

(Step 1) An esterification reaction was conducted by the same method as Example 1.

(Step 2) Stripping was progressed under the same conditions as Example 1, except that the stripping time was changed to 5 minutes, thus obtaining a reaction mixture comprising 2-EH in the content of 5 wt%, based on the total weight of the reaction mixture.

(Step 3) And then, hydrolysis of catalyst and neutralization of acid components were progressed by the same method as Example 1.

(Step 4) And then, using a batch reactor, while flowing nitrogen at 180 °C, 0.05 kg/cm$^2$ at a rate of 500 cc/min, stripping was progressed for 60 minutes to remove residual 2-EH and water.

(Step 5) The reaction mixture, after stripping had been completed, was cooled to 120 °C, and diatomaceous earth was introduced, and then, a filtering process was conducted using a filter paper to prepare dioctyl terephthalate.

### Example 4

Dioctyl terephthalate was prepared by the same method as Example 3, except that the temperature of the neutralization and catalyst hydrolysis step(step 3) was 150 °C, and the pressure was 3 bar.

### Comparative Examples 1 to 3

After the esterification reaction, without primary stripping, distilled water and a neutralization agent were introduced in the reaction mixture to progress hydrolysis of catalyst and neutralization of acid components under conditions of the following Table 1. And then, stripping and filtering processes were conducted by the same method as the steps 4 and 5 of Example 3.

However, in the case of Comparative Examples 1 and 2, due to catalyst residues that had not been hydrolyzed, the filter was blocked during the filtering process, and the filtering process could not be progressed any longer, and thus, pure DOTP could not be obtained.

It was confirmed that the acid values of DOTP prepared through the preparation methods of Examples 1 to 4 and Comparative Example 3 were all 0.01 KOHmg/g or less, and the purities were all 99.0 wt% or more.

[Table 1]

| | 2-EH content after step 2 (wt%) | Conditions for conducting step 3 | | | Whether or not step 5 could be progressed |
| --- | --- | --- | --- | --- | --- |
| | | temperature (°C) | pressure (bar) | Reaction time(min) | |
| Example 1 | 0 | 170 | 5 | 5 | O |
| Example 2 | 0 | 150 | 3 | 5 | O |
| Example 3 | 5 | 170 | 5 | 5 | O |
| Example 4 | 5 | 150 | 3 | 5 | O |
| Comparative Example 1 | 25 | 120 | 1 | 5 | × |
| Comparative Example 2 | 25 | 100 | 1 | 5 | × |
| Comparative Example 3 | 25 | 120 | 1 | 20 | O |

As the results of experiments, in the case of Examples 1 to 4 wherein after the esterification reaction, a process of pre-removing unreacted alcohol from the reaction mixture was conducted, neutralization and hydrolysis of catalyst could be progressed under high temperature and high pressure without a separate cooling process. And, as such, since the process of step 3 was progressed under high temperature and high pressure, the reaction time of hydrolysis of catalyst could be remarkably shortened, and impurities such as catalyst decomposition products (TiO$_2$) and salts, and the like could be easily removed by filtration.

However, in the case of Comparative Examples 1 to 3 wherein the reaction was conducted under low temperature of 100 to 120 °C and atmospheric pressure condition, it was confirmed that significantly long reaction time was required so as to sufficiently decompose the catalyst (Comparative Example 3), and when the reaction time was short, catalyst decomposition could not smoothly occur, and thus, due to aggregated or gelled catalyst and residues, the filter was blocked during filtering (Comparative Examples 1 and 2)

## Claims

1. A method for preparing an ester compound comprising steps of:

   reacting polycarboxylic acid and alcohol in the presence of a titanium-based catalyst to obtain a reaction mixture comprising an ester compound (step 1);
   removing unreacted alcohol from the reaction mixture such that the content of unreacted alcohol becomes 5 wt% or less (step 2);
   adding base and water to the reaction mixture from which unreacted alcohol has been removed, and conducting a neutralization reaction and hydrolysis reaction of the titanium-based catalyst at a pressure of 3 bar or more and a temperature of 150 °C or more (step 3);
   removing water from the reaction mixture in which the neutralization reaction and hydrolysis reaction have been completed (step 4); and
   filtering (step 5).

2. The method for preparing an ester compound according to claim 1, wherein the temperature of the step 2 is 150 °C to 250 °C.

3. The method for preparing an ester compound according to claim 1, wherein the temperature of the step 3 is 150 °C to 190 °C.

4. The method for preparing an ester compound according to claim 1, wherein the pressure of the step 3 is 3 bar to 10 bar.

5. The method for preparing an ester compound according to claim 1, wherein the step 3 is conducted for 10 minutes or less.

6. The method for preparing an ester compound according to claim 1, wherein the base of the step 3 is one or more selected from the group consisting of caustic soda(NaOH), caustic potash(KOH), sodium carbonate($Na_2CO_3$), and sodium bicarbonate.

7. The method for preparing an ester compound according to claim 1, wherein the step 4 is conducted by a decompression stripping process.

8. The method for preparing an ester compound according to claim 1, wherein the polycarboxylic acid is phthalic acid, isophthalic acid, or terephthalic acid.

9. The method for preparing an ester compound according to claim 1, wherein the alcohol is C5-20 aliphatic alcohol.

10. The method for preparing an ester compound according to claim 1, wherein the titanium-based catalyst is one or more selected from the group consisting of tetra isopropyl titanate, tetra n-butyl titanate, tetra octyl titanate and butyl tin maleate.

11. The method for preparing an ester compound according to claim 1, wherein a separate cooling process is not included between the step 1 to step 3.

12. An ester compound prepared by the preparation method according to any one of claims 1 to 11.

13. The ester compound according to claim 12, wherein impurity content is less than 1.0 wt%.

14. The ester compound according to claim 12, wherein acid value is 0.1 KOHmg/g or less.

15. A plasticizer composition comprising the ester compound of claim 12.

## INTERNATIONAL SEARCH REPORT

| International application No. |
|---|
| **PCT/KR2021/001028** |

### A. CLASSIFICATION OF SUBJECT MATTER

**C07C 67/48**(2006.01)i; **C07C 69/612**(2006.01)i; **B01J 31/02**(2006.01)i

According to International Patent Classification (IPC) or to both national classification and IPC

### B. FIELDS SEARCHED

Minimum documentation searched (classification system followed by classification symbols)

C07C 67/48(2006.01); C07C 67/08(2006.01); C07C 67/54(2006.01); C07C 67/60(2006.01); C07C 67/62(2006.01); C07C 69/76(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 폴리카르복실산(polycarboxylic acid), 알코올(alcohol), 티타늄 촉매(titanium catalyst), 에스테르화(esterification), 감압(decompression), 가수분해(hydrolysis), 중화반응(neutralization reaction), 가소제 (plasticizer)

### C. DOCUMENTS CONSIDERED TO BE RELEVANT

| Category* | Citation of document, with indication, where appropriate, of the relevant passages | Relevant to claim No. |
|---|---|---|
| X | KR 10-1990-0008130 B1 (SAMSUNG PETROCHEMICAL CO., LTD.) 31 October 1990 (1990-10-31) See pages 2 and 3; example 1; claim 6; and table 1. | 1-14 |
| Y | | 15 |
| Y | KR 10-2008-0017399 A (EXXONMOBIL CHEMICAL PATENTS INC.) 26 February 2008 (2008-02-26) See claims 1 and 34. | 15 |
| A | KR 10-2017-0037642 A (BASF SE) 04 April 2017 (2017-04-04) See entire document. | 1-15 |
| A | KR 10-2010-0112534 A (DAICEL CHEMICAL INDUSTRIES, LTD.) 19 October 2010 (2010-10-19) See entire document. | 1-15 |

☑ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **10 May 2021** | **10 May 2021** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

## INTERNATIONAL SEARCH REPORT

| | International application No. |
|---|---|
| | **PCT/KR2021/001028** |

**C.     DOCUMENTS CONSIDERED TO BE RELEVANT**

| Category* | Citation of document, with indication, where appropriate, of the relevant passages | Relevant to claim No. |
|---|---|---|
| A | KR 10-0284475 B1 (EXXON CHEMICAL PATENTS INC.) 02 April 2001 (2001-04-02)<br>See entire document. | 1-15 |

Form PCT/ISA/210 (second sheet) (July 2019)

<table>
<tr><td colspan="2">INTERNATIONAL SEARCH REPORT<br>Information on patent family members</td><td colspan="2">International application No.<br><br>**PCT/KR2021/001028**</td></tr>
</table>

| Patent document cited in search report | Publication date (day/month/year) | Patent family member(s) | Publication date (day/month/year) |
|---|---|---|---|
| KR    10-1990-0008130    B1 | 31 October 1990 | KR   10-1989-0009841       A | 04 August 1989 |
| KR    10-2008-0017399    A | 26 February 2008 | JP          2008-542230      A | 27 November 2008 |
|  |  | JP              5374149      B2 | 25 December 2013 |
|  |  | KR          10-1338617      B1 | 06 December 2013 |
|  |  | US          2007-0230698    A1 | 04 October 2007 |
|  |  | US          2008-0275267    A1 | 06 November 2008 |
|  |  | US          2011-0147650    A1 | 23 June 2011 |
|  |  | US              7919649      B2 | 05 April 2011 |
|  |  | US              7987514      B2 | 26 July 2011 |
|  |  | US              8829227      B2 | 09 September 2014 |
|  |  | US              8987499      B2 | 24 March 2015 |
| KR    10-2017-0037642    A | 04 April 2017 | JP          2017-526660      A | 14 September 2017 |
|  |  | US          2017-0267627    A1 | 21 September 2017 |
|  |  | US              9932292      B2 | 03 April 2018 |
| KR    10-2010-0112534    A | 19 October 2010 | JP          2010-241765      A | 28 October 2010 |
| KR        10-0284475    B1 | 02 April 2001 | JP          08-505879        A | 25 June 1996 |
|  |  | KR   10-1996-0700216       A | 19 January 1996 |
|  |  | US              5324853      A | 28 June 1994 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020200018495